# EUROPÄISCHE PATENTSCHRIFT

(11) **EP 3 237 029 B1**
(45) Veröffentlichungstag und Bekanntmachung des Hinweises auf die Patenterteilung: **19.06.2019**
(21) Anmeldenummer: 15820097.2
(22) Anmeldetag: 17.12.2015
(51) Int. Cl.: A61M 1/00, A61J 1/14

(54) **VORRICHTUNG ZUR BEREITSTELLUNG VON UNTERDRUCK FÜR MEDIZINISCHE ANWENDUNGEN**
DEVICE FOR PROVIDING VACUUM FOR MEDICAL APPLICATIONS
DISPOSITIF D'OBTENTION D'UN VIDE POUR APPLICATIONS MÉDICALES

(30) Priorität: 23.12.2014 DE 102014226890
(43) Veröffentlichungstag der Anmeldung: 01.11.2017
(73) Patentinhaber: Paul Hartmann AG, 89522 Heidenheim (DE)
(72) Erfinder: BEYRLE, Karina, 89075 Ulm (DE); CROIZAT, Pierre, 89542 Herbrechtingen (DE); HOFSTETTER, Juergen, 52349 Düren (DE); KLUSMANN, Johanna, 73450 Neresheim (DE); SINCLAIR, John Allan, Cambridge Cambridgeshire CB1 3DT (GB); GRIMWADE, Stephen John, Upwood Huntingdon Cambridgeshire PE26 2Q (GB)
(74) Vertreter: DREISS Patentanwälte PartG mbB
(86) Internationale Anmeldenummer: PCT/EP2015/080233
(87) Internationale Veröffentlichungsnummer: WO 2016/102303

(56) Entgegenhaltungen:
- DE-U1-202013 101 367
- US-A- 5 393 113
- US-A1- 2007 215 782
- US-A1- 2011 265 889
- US-A1- 2013 110 057
- US-A1- 2013 289 536
- US-B1- 6 371 947

## Beschreibung

Die Erfindung betrifft eine Vorrichtung zur Bereitstellung von Unterdruck für medizinische Anwendungen, nämlich zur Unterdruckbehandlung von Wunden am menschlichen oder tierischen Körper, mit einem Gehäuse, das einen ersten Gehäuseteil und einen zweiten Gehäuseteil umfasst, wobei der erste Gehäuseteil eine Unterdruck erzeugende Einrichtung umfasst und der zweite Gehäuseteil einen Behälter zur Aufnahme von Körperflüssigkeiten, insbesondere von aus einer Wunde abgesaugten Wundsekreten, umfasst, und wobei der zweite Gehäuseteil an dem ersten Gehäuseteil lösbar befestigbar ist, und wobei ein Anschluss für eine zum Körper führende Saugleitung vorgesehen ist, so dass eine Unterdruckkommunikation zwischen der Unterdruck erzeugenden Einrichtung, dem Behälter und der zum Körper führenden Saugleitung herstellbar ist. Der Behälter zur Aufnahme von Körperflüssigkeiten ist nach Gebrauch typischerweise wegwerfbar.

Bei derartigen Vorrichtungen zur Unterdruckbehandlung von Wunden kommuniziert eine Unterdruck erzeugende Einrichtung über eine Saugleitung mit der Wunde oder der Wundumgebung. Ein Unterdruck im Wundraum ist dabei über ein luftundurchlässiges Abdeckmaterial zum weitgehend unterdruckdichten Verschließen der Wunde und der Wundumgebung herstellbar. Das luftundurchlässige Abdeckmaterial dichtet den Wundraum derart ab, dass ein Unterdruck im Wundraum herstellbar ist und Flüssigkeiten aus dem Wundraum in den genannten Behälter absaugbar sind.

Mit Unterdruck wird im Zusammenhang mit der vorliegenden Erfindung ein gegenüber dem Umgebungsluftdruck (atmosphärischer Luftdruck) erniedrigter Druck, insbesondere innerhalb eines Wundverbands bezeichnet. Das Abdeckmaterial eines Wundverbands zum luftdichten Verschließen eines Wundraums muss daher so ausgebildet sein, dass es der sich einstellenden Druckdifferenz standzuhalten vermag, damit der Unterdruck im Wundraum überhaupt angelegt und aufrechterhalten werden kann.

Im Bereich der Unterdrucktherapie in der Wundbehandlung wird der Unterdruck quantitativ als Druckdifferenz zwischen dem Umgebungsluftdruck und dem unterhalb des Abdeckmaterials angelegten Luftdruck angegeben. Typischerweise beträgt diese Druckdifferenz im Bereich der Unterdrucktherapie höchstens 250 mm Hg (mm Quecksilbersäule) (1 mm Hg = 1 Torr. = 133,322 Pa). Dieser Unterdruckbereich bis höchstens 250 mm Hg hat sich als für die Wundheilung geeignet erwiesen. Ein bevorzugter Unterdruckbereich liegt zwischen 10 und 150 mm Hg.

Der unter Verwendung der Vorrichtung an die Wunde angelegte Unterdruck kann bei einer typischen Unterdruckbehandlung entweder zeitlich im Wesentlichen konstant gehalten werden, oder er kann zeitlich, insbesondere zyklisch, verändert werden. Die Steuerung bzw. Regelung des angelegten Unterdrucks kann über eine entsprechend ausgebildete und programmierte Steuervorrichtung bei der Unterdruck erzeugenden Einrichtung, insbesondere in Abhängigkeit weiterer Parameter, realisiert werden.

Zum Anlegen von Unterdruck und vorzugsweise auch zum Absaugen von Körperflüssigkeiten ist eine vorzugsweise flexible Saugleitung, vorgesehen, die einenends über einen sogenannten Port im Bereich des Wundabdeckmaterials mit der Wundumgebung oder dem Wundraum und anderenends mit dem eingangs erwähnten Behälter zur Aufnahme von Körperflüssigkeiten bzw. mit der Unterdruck erzeugenden Einrichtung kommuniziert.

Neben der Unterdruckwundbehandlung sind auch andere Anwendungen der hier in Rede stehenden Vorrichtung zur Bereitstellung von Unterdruck für medizinische Anwendungen denkbar, insbesondere die Absaugung an sich beliebiger Körperflüssigkeiten, z.B. im Bereich der medizinischen Inkontinentenversorgung, der Versorgung von Stoma-Patienten oder im Bereich der Absaugung von Wundsekreten, gegebenenfalls unter Verwendung von Spülflüssigkeiten, auch ohne Anlegen eines Unterdrucks über wesentliche Zeiträume.

Die US 2011/265889 A1 beschreibt eine Urinsammelvorrichtung mit einem Tragehenkel. Aus US2013/0289536A1 ist eine Vorrichtung zur Unterdruckbehandlung von Wunden bekannt, welche auf einer Halterung montiert ist.

Der vorliegenden Erfindung liegt die Aufgabe zugrunde, eine betriebssichere und einfach zu realisierende Anbringbarkeit der Vorrichtung an einem Träger, beispielsweise an einer horizontalen Strebe eines Patientenbetts, zu schaffen.

Diese Aufgabe wird bei einer Vorrichtung der eingangs genannten Art erfindungsgemäß dadurch gelöst, dass die Vorrichtung ein formstabiles Halteteil, zum Anbringen der Vorrichtung an einem Träger, insbesondere einer Bettstrebe, einer Stange und/oder einem Gestell, umfasst, welches Halteteil gegenüber dem Gehäuse zwischen einer Gebrauchsstellung, in welcher es wenigstens teilweise von dem Gehäuse abragt, und einer Staustellung, in welcher es wenigstens teilweise, vorzugsweise entlang seiner gesamten Erstreckung, an dem Gehäuse anliegt, verschwenkbar ist und welches Halteteil derart bügelartig ausgebildet ist, dass eine Durchgriffsöffnung entweder durch das Halteteil oder durch das Halteteil und das Gehäuse gebildet und in einer Umfangsrichtung durchgehend begrenzt wird, wenn sich das Halteteil in der Gebrauchsstellung befindet, wobei das Halteteil einen hakenartigen Abschnitt zum einhakenden und/oder anhakenden Anbringen der Vorrichtung an dem Träger aufweist.

Bügelartig im vorliegenden Sinne bedeutet, dass das Halteteil in seiner Gebrauchsstellung und entlang seiner Erstreckung ausgehend von einem gehäuseseitigen Bereich oder Ende des Halteteils vom Gehäuse wegführt und unter Begrenzung der Durchgriffsöffnung wieder zum Gehäuse zurückführt. Dabei kann das Halteteil in sich geschlossen ausgebildet sein und dadurch die Durchgriffssöffnung in Umfangsrichtung durchgehend vollständig begrenzen. Das Halteteil kann auch von einem ersten Befestigungspunkt am Gehäuse wegführen und zu einem zweiten Befestigungspunkt am Gehäuse hinführen, so dass die Durchgriffssöffnung dann durch das Halteteil und das Gehäuse gemeinsam begrenzt wird, also durch das Halteteil und den Bereich des Gehäuses zwischen den beiden Befestigungspunkten. Der erste Befestigungspunkt und der zweite Befestigungspunkt des Halteteils am Gehäuse können dabei auch nahe oder unmittelbar beieinander liegen. Liegen die beiden Befestigungspunkte nahe oder unmittelbar beieinander, so wird die Durchgriffssöffnung wiederum im wesentlichen vollständig durch das Halteteil begrenzt. Vorteilhafterweise jedoch sind der erste Befestigungspunkt und der zweite Befestigungspunkt des Halteteils am Gehäuse voneinander beanstandet, insbesondere auf gegenüberliegenden Seiten des Gehäuses, vorzugsweise des ersten Gehäuseteils, angeordnet. Sie sind weiter vorzugsweise auf horizontal einander gegenüberliegenden Seiten des Gehäuses angeordnet, wenn sich die Vorrichtung in ihrer Gebrauchsstellung befindet.

Das bügelförmige Halteteil kann ganz oder abschnittsweise bogenförmig gekrümmt verlaufen und muss auch nicht in einer Ebene erstreckt sein. Es kann auch abschnittsweise gerade verlaufen, insbesondere einen rechteckförmigen Verlauf aufweisen. Vorzugsweise gehen aneinander anschließende Abschnitte des Halteteils verrundet ineinander über.

Unter einem formstabilen Halteteil ist ein Halteteil zu verstehen, dass seine Form selbstständig oder anders ausgedrückt aufgrund der Stabilität seines Materials und/oder seiner Form beibehält, also Krafteinwirkungen einen gewissen formhaltenden Widerstand entgegensetzt. Dies ist bei im Wesentlichen starren Halteteilen der Fall. Dabei kann jedenfalls auch eine geringfügige, elastisch nachgiebige Auslenkbarkeit des Halteteils unter den Begriff formstabil fallen, etwa derart, dass das Halteteil gerade soweit elastisch aufgedehnt werden kann, dass es am Gehäuse anbringbar ist. Unter "Anbringen" ist im vorliegenden Sinne beispielsweise ein Aufhängen oder Anhängen an den Träger zu verstehen, derart, dass der Träger ein Auflager oder Stützlager für das Halteteil bildet. Ebenso ist hierrunter eine klemmende Befestigung oder eine schnappende, rastende oder in sonstiger Weise hintergreifende Befestigung zu verstehen, die aber lösbar sein muss.

Bei einer Weiterbildung der erfindungsgemäßen Vorrichtung liegt das Halteteil in der Staustellung, vorzugsweise entlang seiner gesamten Erstreckung, an dem ersten Gehäuseteil an. Hierdurch wird erreicht, dass ein Herausragen des Halteteils über eine Außenkontur der Vorrichtung minimiert bzw. komplett vermieden wird, wenn sich das Halteteil in der Staustellung befindet. Das Halteteil soll sich dabei gewissermaßen an die Außenkontur des Gehäuses anschmiegen, wobei das Gehäuse dabei auch zusätzlich eine oder mehrere Ausnehmungen aufweisen kann, in die das Halteteil oder ein Abschnitt des Halteteils eingeschwenkt sein kann.

Durch das Anliegen am Gehäuse bzw. die Einschwenkbarkeit in die Ausnehmungen wird beispielsweise die Gefahr minimiert, dass ein Patient, der die Vorrichtung mit sich führt, mit dem Halteteil an Gegenständen in seiner Umgebung hängen bleibt.

Vorzugsweise ist die Vorrichtung sowohl für den stationären Gebrauch als auch für den mobilen Gebrauch zum Mitführen am Körper des Benutzers ausgebildet und kann hierfür zusätzliche Haltemittel umfassen oder mit solchen zusammenwirken.

Vorzugsweise ist das Halteteil durch Verschwenken um 140 Grad bis 220 Grad, inbesondere um 160 Grad bis 200 Grad, insbesondere um 170 bis 190 Grad oder insbesondere im Wesentlichen um 180°, zwischen der Staustellung und der Gebrauchsstellung verschwenkbar.

Bei einer erfindungsgemäßen Weiterbildung der Vorrichtung ist das Halteteil um eine in einer bestimmungsgemäßen Betriebsposition der Vorrichtung horizontal verlaufende Schwenkachse verschwenkbar, wobei die Schwenkachse vorzugsweise parallel zu einer Bedienebene der Vorrichtung verläuft.

Erfindungsgemäß weist das Halteteil einen hakenartigen Abschnitt zum einhakenden und/oder anhakenden Anbringen der Vorrichtung an dem Träger auf. Hierdurch lässt sich die Vorrichtung in einfacher Weise hängend an einem Träger befestigen.

Vorzugsweise ist der hakenartige Abschnitt rutschhemmend bzw. rutschfest ausgebildet oder weist mindestens ein rutschhemmendes bzw. rutschfestes Element auf, beispielsweise einen rutschhemmenden Kunststoffclip oder Kunststoffmantel.

Im Sinne der Erfindung ist auch, wenn das Halteteil einen metallischen Werkstoff, vorzugsweise Edelstahl, umfasst. Dies ermöglicht unter anderem eine einfache Reinigung des Halteteils, was im vorliegend gegebenen medizinischen Anwendungsbereich von wesentlicher Bedeutung ist.

Vorteilhafterweise ist an wenigstens einem Bereich des Halteteils eine Einschalung aus Kunststoff angebracht, vorzugsweise angeclipst, aufgeclipst, angespritzt oder angegossen. Die Einschalung aus Kunststoff hat vorzugsweise rutschhemmende Wirkung. Denkbar ist ebenso, dass die Einschalung ergonomisch geformt ist, um eine händische Greifbarkeit des Halteteils zu verbessern.

Vorzugsweise ist die Vorrichtung derart ausgeführt, dass das Halteteil klemmend oder schnappend, rastend oder in sonstiger Weise hintergreifend lösbar am Gehäuse gegen Verschwenken gesichert ist, wenn es sich in der Staustellung befindet. Vorzugsweise wirkt ein Teil des hakenartigen Abschnitts mit dem Gehäuse, vorzugsweise dem ersten Gehäuseteil derart zusammen, dass das Halteteil in der Staustellung gegen ein Ausschwenken gesichert ist.

Bei einer bevorzugten Ausführungsform der erfindungsgemäßen Vorrichtung, weist das Halteteil, vorzugsweise im Bereich einer Schwenkachse des Halteteils, mindestens einen Befestigungsabschnitt zum Anbringen eines zusätzlichen, vorzugsweise biegeschlaffen Tragemittels, beispielsweise eines Tragegurts, auf. Unter einem biegeschlaffen Tragemittel ist dabei ein Tragemittel zu verstehen, welches nicht formstabil ist, welches sich also beim Tragen durch einen Patienten dessen Körperform anzupassen vermag.

Vorteilhafterweise ist dabei der Befestigungsabschnitt rutschhemmend ausgebildet und/oder weist rutschhemmende Mittel auf. Hierdurch wird verhindert, dass das vorzugsweise biegeschlaffe Tragemittel entlang des Halteteils verrutscht. Hierbei kann die Struktur des Halteteils selbst das Verrutschen des biegeschlaffen Tragemittels verhindern. Dies kann beispielsweise durch eine bereichsweise ösenartige oder abgewinkelte Ausbildung realisiert werden, aber auch dadurch, dass das Haltemittel bereichsweise derart nahe an dem Gehäuse verläuft, dass das Tragemittel durch einen formschlüssigen Anschlag am Verrutschen gehindert wird. Unter rutschhemmenden Mitteln sind beispielsweise Manschetten zu verstehen, die eine formschlüssige Befestigung des biegeschlaffen Tragemittels ermöglichen, oder eine rutschhemmende Beschichtung des Halteteils.

Vorteilhafterweise ist das Halteteil über eine Schwenkzapfenverbindung mit dem Gehäuse verbunden. Dabei ist es besonders bevorzugt, wenn das Halteteil Schwenkzapfen umfasst, die vorzugsweise mit Kunststoff ummantelt sind. Weiter erweist es sich als vorteilhaft, wenn die Schwenkzapfenverbindung Eingriffsmulden an dem Gehäuse umfasst, die mit Schwenkzapfen an dem Halteteil zusammenwirken, um die Schwenkzapfenverbindung zu bilden.

Vorteilhafterweise sind die Eingriffsmulden am ersten Gehäuseteil ausgebildet.

Es erweist sich als vorteilhaft, wenn das Halteteil von dem Gehäuse lösbar ist. Dann ist die Gehäuseoberfläche im Bereich der Schwenkzapfenverbindung für Reinigungszwecke leicht zugänglich.

Wenn das Halteteil lösbar mit dem Gehäuse verbunden ist, erweist es sich als vorteilhaft, wenn das Halteteil durch geringfügiges, reversibles, elastisches Verformen oder Aufweiten von dem Gehäuse lösbar ist. Hierdurch können das Gehäuse der Vorrichtung und das Halteteil leicht voneinander gelöst und gesondert und somit gründlicher gereinigt werden, um den hohen hygienischen Anforderungen im medizinischen Bereich zu genügen.
Wenn die Vorrichtung so ausgebildet ist, dass ein erster Befestigungspunkt des Halteteils am Gehäuse und ein zweiter Befestigungspunkt des Halteteils am Gehäuse voneinander beanstandet, insbesondere auf gegenüberliegenden Seiten des Gehäuses, vorzugsweise des ersten Gehäuseteils, angeordnet sind, so wird hierdurch beim Anbringen der Vorrichtung an den Träger eine hohe Stabilität erreicht.
Nach einem weiteren Erfindungsgedanken ist das Halteteil derart bügelartig ausgebildet, dass das Gehäuse in die von dem Halteteil oder von dem Halteteil und dem Gehäuse gebildete Durchgriffsöffnung eingreift, wenn sich das Halteteil in der Staustellung befindet.

Vorteilhaft ist auch, wenn das erste Gehäuseteil und/oder das zweite Gehäuseteil insgesamt scheibenförmig ausgebildet ist bzw. sind. Das heißt, ihre jeweilige Breite in horizontaler Richtung und ihre jeweilige Höhe in vertikaler Richtung sind jeweils größer als ihre Tiefe in horizontaler Richtung und senkrecht zur Breitenerstreckung. Hierdurch ist es möglich, dass die Vorrichtung in Tiefenrichtung insgesamt so ausgebildet und bemessen werden kann, dass sie bequem am Körper eines Benutzers getragen werden kann.

In vorteilhafter Weiterbildung der Vorrichtung liegen das erste Gehäuseteil und das zweite Gehäuseteil über eine, in der bestimmungsgemäßen Betriebsposition der Vorrichtung vorzugsweise im Wesentlichen vertikale, Trennebene gegeneinander an, wobei der Begriff der Ebene hier nicht im exakt geometrischen Sinn zu verstehen ist sondern die gegeneinander anliegenden Seiten der Gehäuseteile ineinandergreifende Vorsprünge und Ausnehmungen aufweisen können.

Es wird ferner selbständiger Schutz in Anspruch genommen für eine Vorrichtung mit den Merkmalen des Oberbegriffs des Anspruchs 1, die weiter ein formstabiles Halteteil zum Anbringen der Vorrichtung an einem Träger, insbesondere einer Bettstrebe, einer Stange und/oder einem Gestell, aufweist, welches Halteteil gegenüber dem Gehäuse zwischen einer Gebrauchsstellung, in welcher es wenigstens teilweise von dem Gehäuse abragt, und einer Staustellung, in welcher es wenigstens teilweise, vorzugsweise entlang seiner gesamten Erstreckung, an dem Gehäuse anliegt, verschwenkbar ist, wobei das Halteteil um eine in einer bestimmungsgemäßen Betriebsposition der Vorrichtung horizontal verlaufende Schwenkachse zwischen der Gebrauchsstellung und der Staustellung verschwenkbar ist, wobei das Halteteil einen hakenartigen Abschnitt zum einhakenden und/oder anhakenden Anbringen der Vorrichtung an dem Träger aufweist. Es wird ferner Schutz in Anspruch genommen für eine Weiterbildung dieser Vorrichtung mit den Merkmalen der Unteransprüche jeweils einzeln und in beliebiger Kombination.

Weitere Einzelheiten, Merkmale und Vorteile der Erfindung ergeben sich aus den beigefügten Patentansprüchen und aus der zeichnerischen Darstellung und nachfolgenden Beschreibung bevorzugter Ausführungsformen der Erfindung. In der Zeichnung zeigen:
- Figuren 1a bis d: verschiedene Ansichten einer erfindungsgemäßen Ausführungsform einer am Körper tragbaren Vorrichtung zur Bereitstellung von Unterdruck für medizinische Anwendungen mit einem Halteteil in einer Gebrauchsstellung;
- Figuren 2: Vorrichtung nach Figur 1 mit dem Halteteil in einer Staustellung;
- Figuren 3a bis d: verschiedene Ansichten eines eine Unterdruck erzeugende Einrichtung und Steuerungskomponenten umfassenden ersten Gehäuseteils einer alternativen Ausführungsform der erfindungsgemäßen Vorrichtung mit einem Halteteil in der Staustellung;
- Figuren 4a bis c: verschiedene Ansichten des Halteteils der Vorrichtung nach Figur 3;
- Figur 5: eine Detailansicht eines Teilbereichs der Vorrichtung nach Figur 1; und

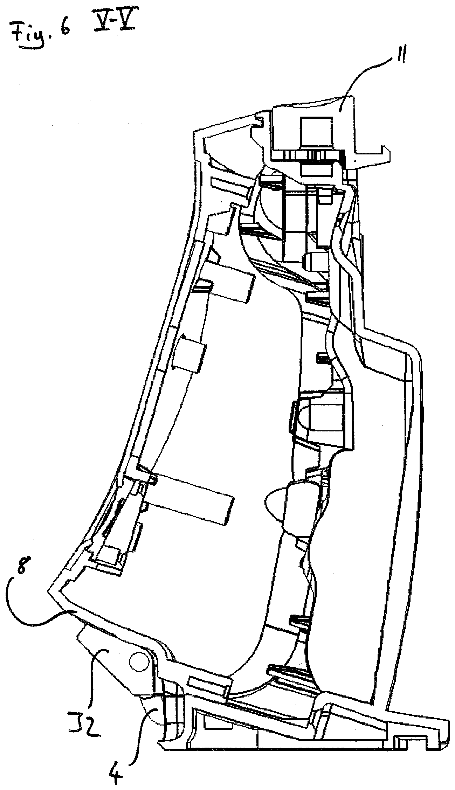

- Figur 6: eine Schnittansicht entlang der Linie V-V der Figur 3c der Vorrichtung nach Figur 3.

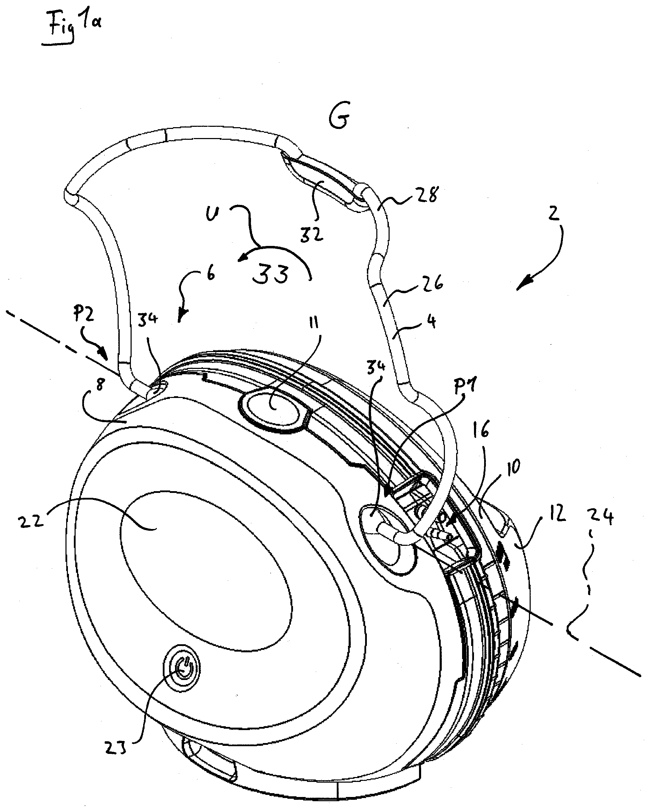

Die Figuren 1a bis 1d zeigen eine erfindungsgemäße Ausführungsform einer tragbaren Vorrichtung 2 zur Bereitstellung von Unterdruck für medizinische Anwendungen. Die Vorrichtung 2 weist ein formstabiles später im Einzelnen zu erläuterndes Halteteil 4 auf, das in den Figuren 1a bis 1d in einer Gebrauchsstellung G gezeigt ist.

Die Vorrichtung 2 umfasst ein Gehäuse 6 mit einem ersten Gehäuseteil 8, in dem eine Unterdruck erzeugende Einrichtung beispielsweise in Form einer Luftpumpe angeordnet ist. Im ersten Gehäuseteil 8 sind auch elektrische und elektronische Steuerkomponenten für die Vorrichtung insgesamt, einschließlich Batterien oder vorzugsweise wiederaufladbarer Akkus aufgenommen.

Die Vorrichtung 2 umfasst ein zweites Gehäuseteil 10, welches im bevorzugten Fall zugleich einen Behälter 12 zur Aufnahme von Körperflüssigkeiten, insbesondere zur Aufnahme von aus einer Wunde abgesaugten Wundsekreten, bildet. Bevorzugterweise ist der gesamte zweite Gehäuseteil 10 als wegwerfbarer Einmalartikel ausgebildet.

Das zweite Gehäuseteil 10 ist lösbar an dem ersten Gehäuseteil 8 befestigt. Im verbundenen Zustand sind das erste Gehäuseteil 8 und das zweite Gehäuseteil 10 über einen Rastmechanismus 11 gegen Trennen gesichert.

Die beiden Gehäuseteile 8 und 10 sind insgesamt scheibenförmig ausgebildet, das heißt ihre jeweilige Breite B8, B10 in horizontaler Richtung und ihre jeweilige Höhe H8, H10 in vertikaler Richtung sind jeweils größer als ihre Tiefe T8, T10 in horizontaler Richtung und senkrecht zur Breitenerstreckung. Hierdurch ist es möglich, dass die Vorrichtung 2 in Tiefenrichtung insgesamt so ausgebildet und bemessen werden kann, dass sie auch stabil am Körper eines Benutzers getragen werden kann.

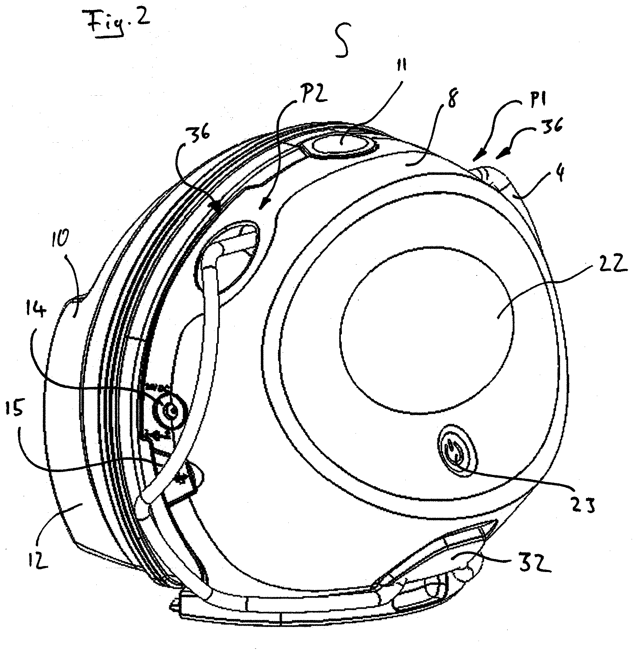

An dem ersten Gehäuseteil 8 ist ein Ladeanschluss 14 für die Akkus vorgesehen. Der Ladeanschluss 14 und ein USB-Anschluss 15 sind in Fig. 2 gezeigt.

Figur 2 zeigt die Vorrichtung 2 mit dem Halteteil 4 in einer Staustellung S. In der Staustellung S liegt das Halteteil 4 vorzugsweise entlang seiner gesamten Erstreckung an dem ersten Gehäuseteil 8 an. In Figur 2 ist ersichtlich, dass das Halteteil 4 derart geformt ist, dass es den Ladeanschluss 14 nicht überdeckt, wenn es sich in der Staustellung S befindet. Dies wird durch einen vom zweiten Gehäuseteil 10 weggekrümmten bogenförmigen Verlauf des Halteteils 4 im Bereich um den Ladeanschluss 14 herum erreicht.

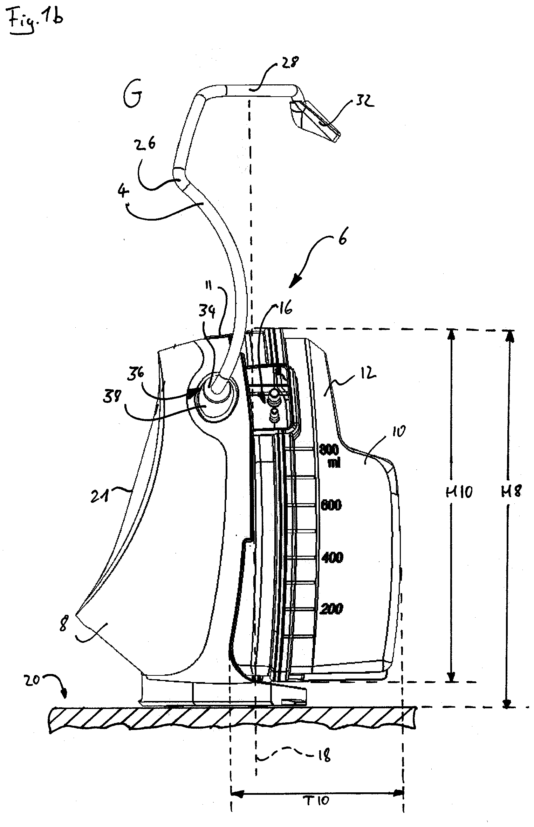

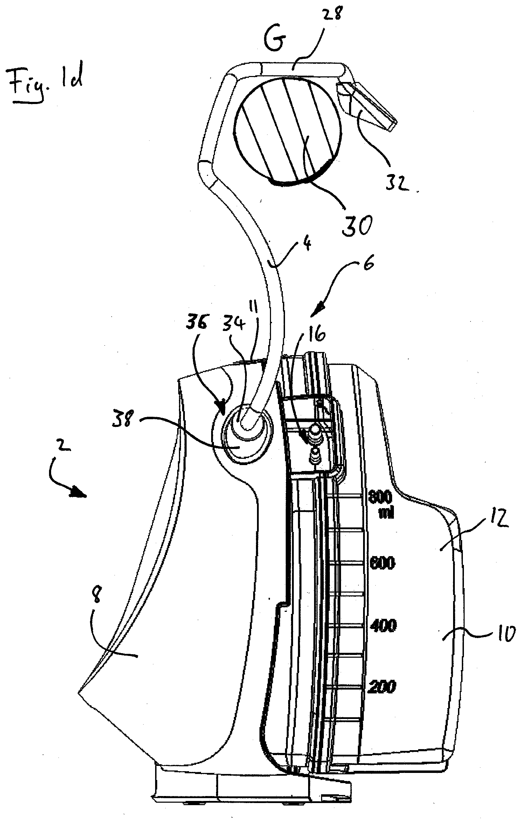

In den Figuren 1a, b und d ist gezeigt, dass an der Vorrichtung 2, und zwar an dem zweiten Gehäuseteil 10 ein Anschluss 16 in Form zweier Anschlussstutzen für eine nicht dargestellte Saugleitung vorgesehen ist. Die Saugleitung führt dann beispielsweise bei der Verwendung der Vorrichtung 2 zur Unterdrucktherapie von Wunden zu einem die Wunde druckdicht verschließenden Wundverband und kommuniziert dort, beispielsweise über einen Port, mit dem Wundraum, um im Wundraum einen Unterdruck anzulegen und aufrechtzuerhalten und Wundsekrete in den Behälter 12 abzusaugen. Über einen der Anschlussstutzen kann beispielsweise ein fluides Medium zugeführt werden.

Im bevorzugten dargestellten Fall liegen das erste Gehäuseteil 8 und das zweite Gehäuseteil 10 über eine im Wesentlichen vertikale Trennebene 18, die in Figur 1b angedeutet ist, gegeneinander an. Wenn die Vorrichtung 2 wie in Figur 1b angedeutet, auf einer ebenen horizontalen Unterlage 20 abgestellt ist, so ist die Trennebene 18 im Wesentlichen vertikal orientiert. Der vertikale Verlauf der Trennebene 18 bedeutet, dass der erste Gehäuseteil 8 und der zweite Gehäuseteil 10 nicht ineinander eingesetzt oder übereinander gestapelt sind, sondern dass sie horizontal nebeneinander im bestimmungsgemäß zusammengesetzten Zustand der Vorrichtung 2 angeordnet sind.

Auf einer Bedienebene 21 können Ausgabeeinrichtungen 22 und Bedienelemente 23 angeordnet sein. Vorliegend ist ein Touchscreen, der bifunktional als Ausgabeeinrichtung 22 und Bedienelement 23 dient, und ein weiteres Bedienelement 23, das als An/Aus-Knopf ausgeführt ist, vorgesehen.

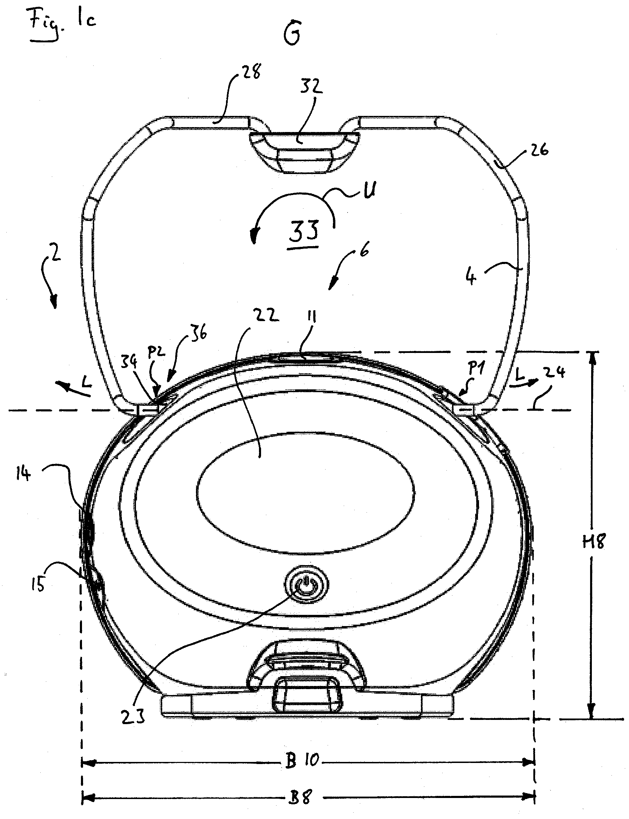

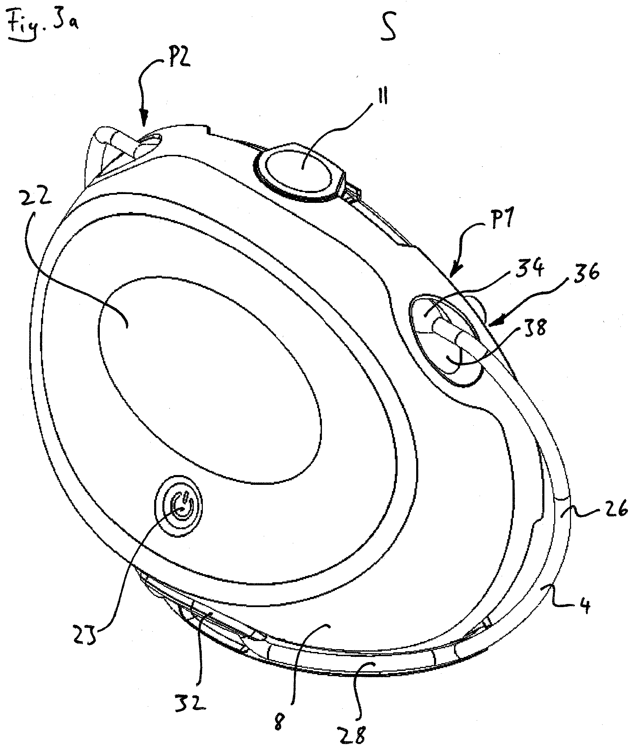

Figuren 3a bis 3d zeigen eine alternative Ausführungsform der Vorrichtung 2, welche sich von der in den Figuren 1 a-d und 2 gezeigten durch die Form des Halteteils 4 unterscheidet.

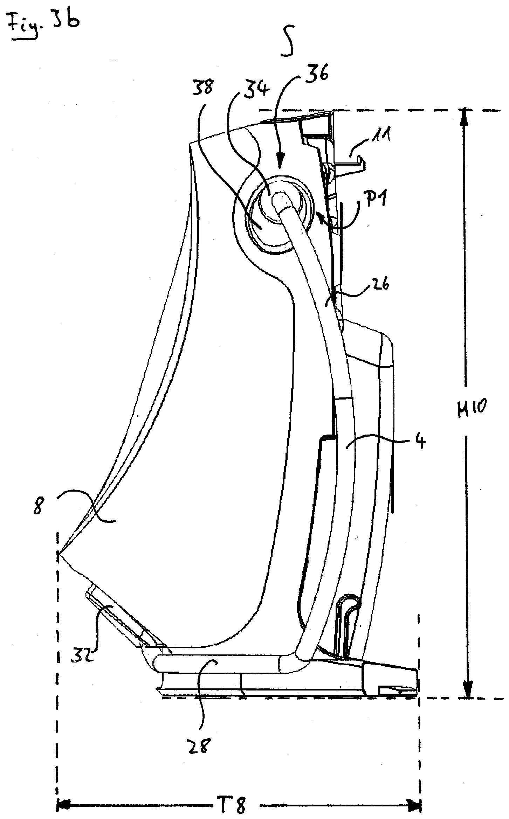

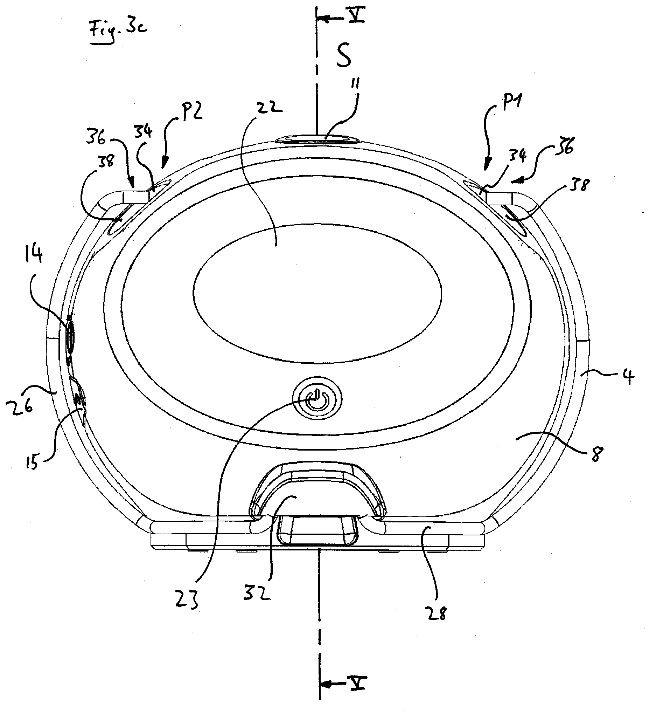

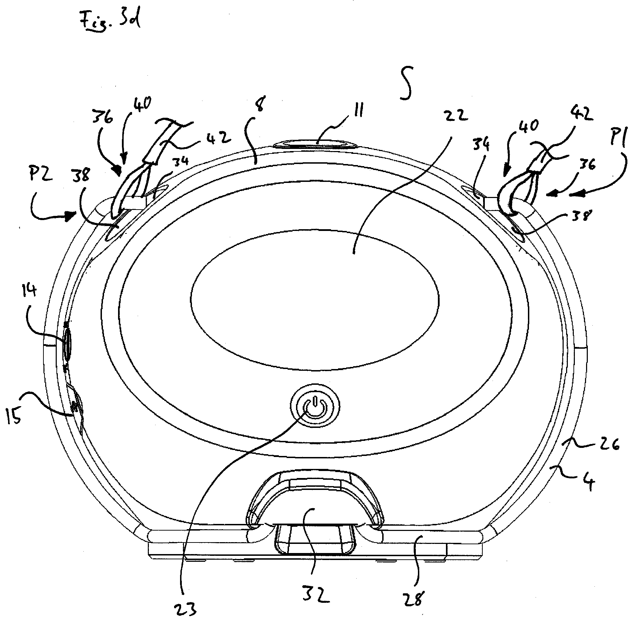

Sowohl das Halteteil 4 nach den Figuren 1 a-d und 2 als auch das Halteteil nach den Figuren 3 a-d ist durch Verschwenken um beispielsweise im Wesentlichen 180° um eine Schwenkachse 24 zwischen der Staustellung S und der Gebrauchsstellung G verschwenkbar. In der bestimmungsgemäßen Betriebsposition der Vorrichtung 2 verläuft die Schwenkachse 24 im Wesentlichen horizontal und parallel zu der Bedienebene 21.

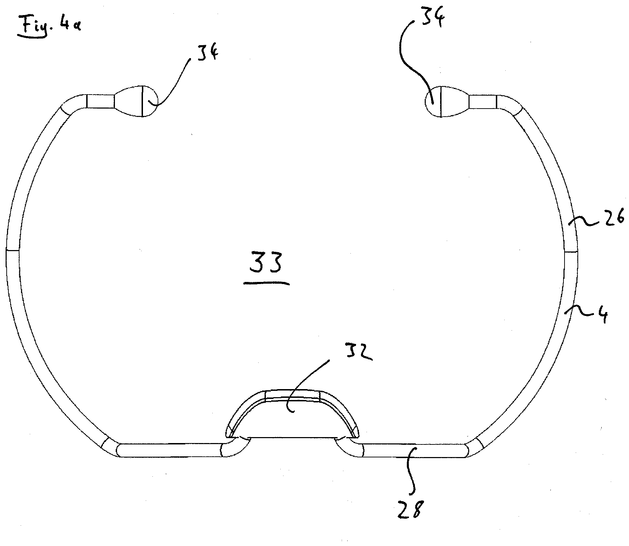

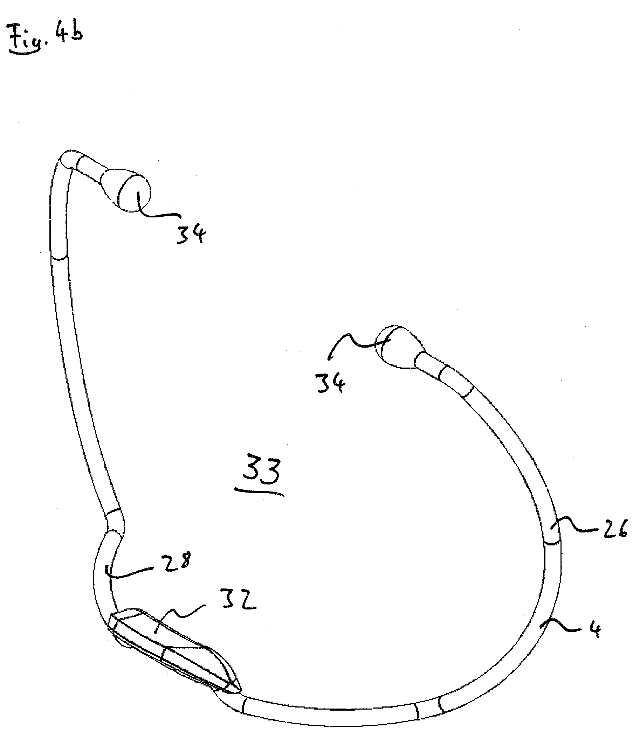

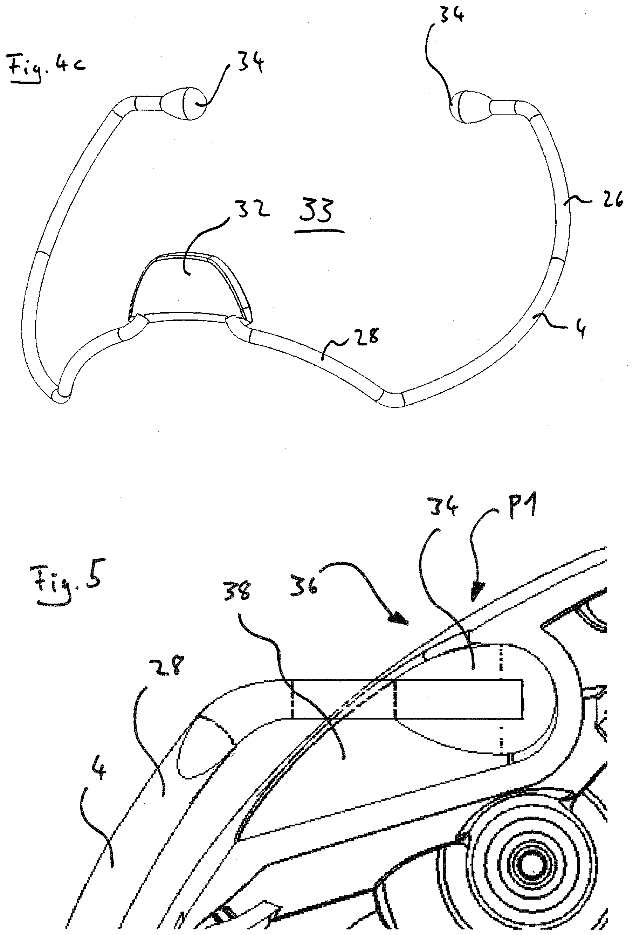

Das Halteteil 4 nach den Figuren 3 a-d, ist in den Figuren 4a bis 4c in abgelöstem Zustand gezeigt. Das Halteteil 4 umfasst einen bügelartigen Rahmen 26, welcher vorzugsweise aus Edelstahl gebildet ist und entlang seiner gesamten Erstreckung einstückig verläuft. Dies gilt auch für das Halteteil 4 nach Figuren 1 und 2.

Das Halteteil 4 beider Ausführungsformen weist einen hakenartigen Abschnitt 28 auf. Wenn sich das Halteteil 4 in der Gebrauchsstellung G befindet, dient der hakenartige Abschnitt 28 zum einhakenden und/oder anhakenden Anbringen der Vorrichtung 2 an einem, in Figur 1d in Form einer Strebe dargestellten, Träger 30.

An dem hakenartigen Abschnitt 28 des aus Edelstahl gebildeten Rahmens 26 ist eine Einschalung 32 aus Kunststoff angebracht, im vorliegenden Fall angegossen. Die Einschalung 32 wirkt in der Gebrauchsstellung G rutschhemmend und erhöht dadurch die Sicherheit und Stabilität der hängenden Anbringung der Vorrichtung 2 mittels des Halteteils 4 an dem Träger 30. In vorliegender Ausführungsform bewirkt die Einschalung 32 auch eine rastende Sicherung des Halteteils 4 an dem ersten Gehäuseteil 8, wenn das Halteteil 4 sich in der Staustellung S befindet.

Das Halteteil 4 erstreckt sich von einem ersten Befestigungspunkt P1 am Gehäuse 6 zu einem zweiten Befestigungspunkt P2 am Gehäuse 6. Der erste Befestigungspunkt P1 ist dabei zu dem zweiten Befestigungspunkt P2 beabstandet und vorliegend an gegenüberliegenden Seiten des ersten Gehäuseteils 8 angeordnet.

Das Halteteil 4 beider Ausführungsformen ist derart bügelartig ausgebildet und am Gehäuse angelenkt, dass es zusammen mit dem Gehäuse 6 eine Durchgriffsöffnung 33 begrenzt, wenn sich das Halteteil 4 in der Gebrauchsstellung G befindet. Diese Durchgriffsöffnung 33 ist in einer Umfangsrichtung, die in Fig. 1a,c durch einen Pfeil mit Bezugszeichen U gekennzeichnet ist, vollständig oder durchgehend begrenzt; sie ist also in Umfangsrichtung U vollständig umschlossen.

Befindet sich das Halteteil in der Staustellung S, so greift das Gehäuse 6 in diese Durchgriffsöffnung 33 ein oder anders ausgedrückt ist das Halteteil über den Außenumfang des Gehäuses geschwenkt.

Das Halteteil 4 weist an beiden Enden seines Verlaufs Schwenkzapfen 34 auf, welche vorliegend mit Kunststoff ummantelt sind. Das Halteteil 4 ist über eine Schwenkzapfenverbindung 36 mit dem Gehäuse 6 verbunden. Die Schwenkzapfenverbindung 36 wird durch Zusammenwirken der Schwenkzapfen 34 am Halteteil 4 und Eingriffsmulden 38 an dem Gehäuse 6, vorliegend am ersten Gehäuseteil 8, gebildet. Die Schwenkzapfenverbindung 36 ist in Figur 5, die einen Bereich um eine der Eingriffsmulden 38 des ersten Gehäuseteils 8 zeigt, vergrößert dargestellt.

Durch geringfügiges, reversibles, elastisches Verformen des Halteteils 4 ist es von dem Gehäuse 6 lösbar. Zum Lösen des Halteteils 4 von dem Gehäuse 6 wird das Halteteil 4 bei der beispielhaft dargestellten Anwendung also derart geringfügig aufgebogen oder aufgedehnt, dass die Schwenkzapfen 34 sich voneinander wegbewegen. Das Aufdehnen ist in Figur 1c durch Pfeile mit Bezugszeichen L angedeutet. Durch das geringfügige, reversible, elastische Verformen des Halteteils 4 und das Voneinanderwegbewegen der Schwenkzapfen 34 werden die Schwenkzapfen 34 aus den Eingriffsmulden 38 am ersten Gehäuseteil 8 bewegt und dadurch das Halteteil 4 vom Gehäuse 6 gelöst.

Die beiden Fortführungen der Schwenkzapfen 34 im Bereich der horizontalen Schwenkachse 24 des Halteteils 4 bilden jeweils einen Befestigungsabschnitt 40 zum Anbringen eines in Figur 3d teilweise dargestellten Tragegurts 42, also eines biegeschlaffen Tragemittels 42. Dabei ist das Halteteil 4 im Bereich der Befestigungsabschnitte 40 derart ausgebildet, dass der Tragegurt 42 am Verrutschen gehindert wird, insbesondere auch durch ein Zusammenwirken des Halteteils 4 mit dem Gehäuse 6.

## Patentansprüche

1. Vorrichtung (2) zur Bereitstellung von Unterdruck für medizinische Anwendungen, nämlich zur Unterdruckbehandlung von Wunden am menschlichen oder tierischen Körper, mit einem Gehäuse (6), das einen ersten Gehäuseteil (8) und einen zweiten Gehäuseteil (10) umfasst,
wobei der erste Gehäuseteil (8) eine Unterdruck erzeugende Einrichtung umfasst und der zweite Gehäuseteil (10) einen Behälter (12) zur Aufnahme von Körperflüssigkeiten, insbesondere von aus einer Wunde abgesaugten Wundsekreten, umfasst,
und wobei der zweite Gehäuseteil (10) an dem ersten Gehäuseteil (8) lösbar befestigbar ist,
und wobei ein Anschluss (16) für eine zum Körper führende Saugleitung vorgesehen ist, so dass eine Unterdruckkommunikation zwischen der Unterdruck erzeugenden Einrichtung, dem Behälter (12) und der zum Körper führenden Saugleitung herstellbar ist,
**gekennzeichnet, durch**
ein formstabiles Halteteil (4) zum Anbringen der Vorrichtung (2) an einem Träger (30), insbesondere einer Bettstrebe, einer Stange und/oder einem Gestell,
welches Halteteil (4) gegenüber dem Gehäuse (6) zwischen einer Gebrauchsstellung (G), in welcher es wenigstens teilweise von dem Gehäuse (6) abragt, und einer Staustellung (S), in welcher es wenigstens teilweise, vorzugsweise entlang seiner gesamten Erstreckung, an dem Gehäuse (6) anliegt, verschwenkbar ist und
welches Halteteil (4) derart bügelartig ausgebildet ist, dass eine Durchgriffsöffnung (33) entweder **durch** das Halteteil (4) oder **durch** das Halteteil (4) und das Gehäuse (6) gebildet und in einer Umfangsrichtung (U) durchgehend begrenzt wird, wenn sich das Halteteil (4) in der Gebrauchsstellung (G) befindet, wobei das Halteteil (4) einen hakenartigen Abschnitt (28) zum einhakenden und/oder anhakenden Anbringen der Vorrichtung (2) an dem Träger (30) aufweist.

2. Vorrichtung (2) nach Anspruch 1, **dadurch gekennzeichnet, dass** wenigstens ein Abschnitt des Halteteils (4) in Ausnehmungen am Gehäuse (6) eingeschwenkt ist, wenn sich das Halteteil (4) in der Staustellung (S) befindet.

3. Vorrichtung (2) nach einem der vorangegangenen Ansprüche, **dadurch gekennzeichnet, dass** das Halteteil (4) um 140 Grad bis 220 Grad, insbesondere um 160 Grad bis 200 Grad, insbesondere um 170 bis 190 Grad, zwischen der Staustellung (S) und der Gebrauchsstellung (G) verschwenkbar ist.

4. Vorrichtung (2) nach einem der vorangegangenen Ansprüche, **dadurch gekennzeichnet, dass** der hakenartige Abschnitt (28) rutschhemmend bzw. rutschfest ausgebildet ist und insbesondere ein rutschhemmendes und/oder rutschfestes Element, insbesondere einen rutschhemmenden Kunststoffclip oder Kunststoffmantel, aufweist.

5. Vorrichtung (2) nach einem der vorangegangenen Ansprüche, **dadurch gekennzeichnet, dass** das Halteteil (4) einen metallischen Werkstoff, insbesondere Edelstahl, umfasst.

6. Vorrichtung (2) nach einem der vorangegangenen Ansprüche, **dadurch gekennzeichnet, dass** an wenigstens einem Bereich des Halteteils (4) eine Einschalung (32) aus Kunststoff angebracht, insbesondere angeclipst, aufgeclipst, angespritzt oder angegossen, ist.

7. Vorrichtung (2) nach einem der vorangegangenen Ansprüche, **dadurch gekennzeichnet, dass** das Halteteil (4) klemmend oder schnappend, rastend oder in sonstiger Weise hintergreifend lösbar am Gehäuse (6) gegen Verschwenken gesichert oder sicherbar ist, wenn es sich in der Staustellung (S) befindet.

8. Vorrichtung (2) nach einem der vorangegangenen Ansprüche, **dadurch gekennzeichnet, dass** das Halteteil (4), vorzugsweise im Bereich einer Schwenkachse (24) des Halteteils (4), mindestens einen Befestigungsabschnitt (40) zum Anbringen eines zusätzlichen, biegeschlaffen Tragemittels (42), beispielsweise eines Tragegurts (42), aufweist.

9. Vorrichtung (2) nach Anspruch 8, **dadurch gekennzeichnet, dass** der Befestigungsabschnitt (40) rutschhemmend ausgebildet ist und/oder rutschhemmende Mittel (32) aufweist.

10. Vorrichtung (2) nach einem der vorangegangenen Ansprüche, **dadurch gekennzeichnet, dass** das Halteteil (4) über eine Schwenkzapfenverbindung (36) mit dem Gehäuse (6) verbunden ist.

11. Vorrichtung (2) nach Anspruch 10, **dadurch gekennzeichnet, dass** das Halteteil (4) Schwenkzapfen (34), die mit Kunststoff ummantelt sind, umfasst.

12. Vorrichtung (2) nach Anspruch 10 und/oder Anspruch 11, **dadurch gekennzeichnet, dass** die Schwenkzapfenverbindung (36) Eingriffsmulden (38) an dem Gehäuse (6) umfasst, die mit Schwenkzapfen (34) an dem Halteteil (4) zusammenwirken, um die Schwenkzapfenverbindung (36) zu bilden.

13. Vorrichtung (2) nach einem der vorangegangenen Ansprüche, **dadurch gekennzeichnet, dass** das Halteteil (4) durch geringfügiges, reversibles, elastisches Verformen von dem Gehäuse (6) lösbar ist.

14. Vorrichtung (2) nach einem der vorangegangenen Ansprüche, **dadurch gekennzeichnet, dass** das Halteteil (4) derart bügelartig ausgebildet ist, dass das Gehäuse (6) in die von dem Halteteil (4) oder von dem Halteteil (4) und dem Gehäuse (6) gebildete Durchgriffsöffnung (33) eingreift, wenn sich das Halteteil (4) in der Staustellung (S) befindet.

15. Vorrichtung mit den Merkmalen des Oberbegriffs des Anspruchs 1, **gekennzeichnet durch** ein formstabiles Halteteil zum Anbringen der Vorrichtung an einem Träger, insbesondere einer Bettstrebe, einer Stange und/oder einem Gestell, welches Halteteil gegenüber dem Gehäuse zwischen einer Gebrauchsstellung, in welcher es wenigstens teilweise von dem Gehäuse abragt, und einer Staustellung, in welcher es wenigstens teilweise, vorzugsweise entlang seiner gesamten Erstreckung, an dem Gehäuse anliegt, verschwenkbar ist, und weiter **dadurch** gekennzeichnet, dass das Halteteil um eine in einer bestimmungsgemäßen Betriebsposition der Vorrichtung horizontal verlaufende Schwenkachse zwischen der Gebrauchsstellung und der Staustellung verschwenkbar ist, wobei das Halteteil (4) einen hakenartigen Abschnitt (28) zum einhakenden und/oder anhakenden Anbringen der Vorrichtung (2) an dem Träger (30) aufweist.

## Claims

1. Device (2) for providing a vacuum for medical uses, specifically for vacuum treatment of wounds on the human or animal body, the device having a housing (6) which comprises a first housing part (8) and a second housing part (10),
the first housing part (8) comprising a vacuum generating apparatus, and the second housing part (10) comprising a container (12) for receiving bodily fluids, in particular wound secretions aspirated from a wound,
and it being possible to detachably attach the second housing part (10) to the first housing part (8),
and a connection (16) being provided for a suction line that leads to the body, such that a vacuum communication can be established between the vacuum generating apparatus, the container (12) and the suction line which leads to the body,
**characterized by**
a dimensionally stable holding part (4) for mounting the device (2) on a support (30), in particular a bed strut, a bar and/or a frame,
which holding part (4) can pivot in relation to the housing (6) between a use position (G), in which it projects at least in part from the housing (6), and a stowage position (S), in which it abuts the housing (6) at least in part, preferably along its entire extension, and
which holding part (4) is bow-shaped in such a way that a reach-through opening (33) is formed and continuously delimited in a peripheral direction (U) either by the holding part (4) or by the holding part (4) and the housing (6) when the holding part (4) is in the use position (G), the holding part (4) having a hook-like portion (28) for mounting the device (2) by hooking into and/or hooking onto the support (30).

2. Device (2) according to claim 1, **characterized in that** at least one portion of the holding part (4) is pivoted into recesses on the housing (6) when the holding part (4) is in the stowage position (S).

3. Device (2) according to either of the preceding claims, **characterized in that** the holding part (4) can be pivoted by 140 to 220 degrees, in particular by 160 to 200 degrees, in particular by 170 to 190 degrees, between the stowage position (S) and the use position (G).

4. Device (2) according to any of the preceding claims, **characterized in that** the hook-like portion (28) is slip-resistant or non-slip, and in particular has a slip-resistant and/or non-slip element, in particular a slip-resistant plastics clip or plastics covering.

5. Device (2) according to any of the preceding claims, **characterized in that** the holding part (4) comprises a metal material, in particular stainless steel.

6. Device (2) according to any of the preceding claims, **characterized in that** a casing (32) made of plastics material is mounted on, in particular clipped to, clipped on, injection-molded on or casted on, at least one region of the holding part (4).

7. Device (2) according to any of the preceding claims, **characterized in that** the holding part (4), when it is in the stowage position (S), is or can be detachably secured on the housing (6) against pivoting by means of clamping or snapping, latching or engaging in another manner.

8. Device (2) according to any of the preceding claims, **characterized in that** the holding part (4), preferably in the region of a pivot axis (24) of the holding part (4), has at least one attachment portion (40) for mounting an additional flexible carrying means (42), for example a carrying strap (42).

9. Device (2) according to claim 8, **characterized in that** the attachment portion (40) is slip-resistant and/or comprises slip-resistant means (32).

10. Device (2) according to any of the preceding claims, **characterized in that** the holding part (4) is connected to the housing (6) by means of a pivot pin connection (36) .

11. Device (2) according to claim 10, **characterized in that** the holding part (4) comprises pivot pins (34) which are encased in plastics material.

12. Device (2) according to claim 10 and/or claim 11, **characterized in that** the pivot pin connection (36) comprises engagement troughs (38) on the housing (6) which cooperate with the pivot pins (34) on the holding part (4) to form the pivot pin connection (36).

13. Device (2) according to any of the preceding claims, **characterized in that** the holding part (4) can be detached from the housing (6) by means of minor, reversible, elastic deformation.

14. Device (2) according to any of the preceding claims, **characterized in that** the holding part (4) is bow-shaped in such a way that, when the holding part (4) is in the stowage position (S), the housing (6) engages into the reach-through opening (33) formed by the holding part (4) or by the holding part (4) and the housing (6).

15. Device comprising the features of the preamble of claim 1, **characterized by** a dimensionally stable holding part for mounting the device on a support, in particular a bed strut, a bar, and/or a frame, which holding part can pivot relative to the housing between a use position, in which it projects at least in part from the housing, and a stowage position, in which it abuts the housing at least in part, preferably along its entire extension, and further **characterized in that** the holding part can be pivoted between the use position and the stowage position about a pivot axis that extends horizontally when the device is in an operating position as intended, the holding part (4) comprising a hook-like portion (28) for mounting the device (2) by hooking into and/or hooking onto the support (30).

## Revendications

1. Dispositif (2) destiné à fournir de la pression négative pour des applications médicales, à savoir pour le traitement par pression négative de plaies sur le corps humain ou animal, comprenant un boîtier (6) qui comprend une première partie de boîtier (8) et une deuxième partie de boîtier (10),
dans lequel la première partie de boîtier (8) comprend un dispositif générant de la pression négative et la deuxième partie de boîtier (10) comprend un récipient (12) destiné à recevoir des fluides corporels, en particulier des sécrétions extraites d'une plaie,
et dans lequel la deuxième partie de boîtier (10) peut être fixée de manière amovible à la première partie de boîtier (8),
et dans lequel un raccord (16) pour une conduite d'aspiration menant vers le corps est prévu de sorte que l'on puisse établir une communication à pression négative entre ledit dispositif générant de la pression négative, ledit récipient (12) et ladite conduite d'aspiration menant vers le corps,
**caractérisé par**
une partie de maintien (4) de forme stable destinée à monter le dispositif (2) sur un support (30), en particulier une entretoise de lit, une tige et/ou un cadre,
laquelle partie de maintien (4) peut pivoter par rapport au boîtier (6) entre une position d'utilisation (G) dans laquelle elle fait saillie au moins en partie du boîtier (6) et une position repliée (S) dans laquelle elle est au moins en partie, de préférence le long de l'ensemble de son extension, en appui sur le boîtier (6), et
laquelle partie de maintien (4) est conçue du type arceau de telle sorte qu'une ouverture traversante (33) est formée soit par la partie de maintien (4) soit par la partie de maintien (4) et le boîtier (6) et est délimitée de façon continue dans une direction circonférentielle (U) lorsque la partie de maintien (4) se trouve dans la position d'utilisation (G), la partie de maintien (4) présentant une portion de type crochet (28) pour un montage accrochant et/ou agrafant du dispositif (2) sur le support (30).

2. Dispositif (2) selon la revendication 1, **caractérisé par le fait qu'**au moins une portion de la partie de maintien (4) est introduite par pivotement dans des évidements sur le boîtier (6) lorsque la partie de maintien (4) se trouve dans la position repliée (S).

3. Dispositif (2) selon l'une quelconque des revendications précédentes, **caractérisé par le fait que** la partie de maintien (4) peut pivoter de 140 degrés à 220 degrés, en particulier de 160 degrés à 200 degrés, en particulier de 170 degrés à 190 degrés, entre la position repliée (S) et la position d'utilisation (G).

4. Dispositif (2) selon l'une quelconque des revendications précédentes, **caractérisé par le fait que** la portion de type crochet (28) est conçue de manière à être antidérapante ou bien à ne pas glisser et, en particulier, présente un élément antidérapant et/ou antiglissant, en particulier un clip en matière plastique ou une gaine en matière plastique antidérapant(e).

5. Dispositif (2) selon l'une quelconque des revendications précédentes, **caractérisé par le fait que** la partie de maintien (4) comprend un matériau métallique, en particulier de l'acier inoxydable.

6. Dispositif (2) selon l'une quelconque des revendications précédentes, **caractérisé par le fait qu'**une enveloppe (32) en matière plastique est fixée à au moins une zone de la partie de maintien (4), en particulier attachée par clipsage, clipsée sur celle-ci, moulée par injection ou surmoulée.

7. Dispositif (2) selon l'une quelconque des revendications précédentes, **caractérisé par le fait que**, lorsque la partie de maintien (4) se trouve dans la position repliée (S), elle est bloquée ou peut être bloquée de manière amovible sur le boîtier (6) contre un pivotement, soit par serrage ou encliquetage, par enclenchement soit d'une autre manière en s'engageant derrière.

8. Dispositif (2) selon l'une quelconque des revendications précédentes, **caractérisé par le fait que** la partie de maintien (4), de préférence au niveau d'un axe de pivotement (24) de la partie de maintien (4), présente au moins une portion de fixation (40) destinée à fixer un moyen de support (42) supplémentaire de forme instable, par exemple une sangle de transport (42).

9. Dispositif (2) selon la revendication 8, **caractérisé par le fait que** la portion de fixation (40) est conçue de manière à être antidérapante et/ou comprend des moyens (32) antidérapants.

10. Dispositif (2) selon l'une quelconque des revendications précédentes, **caractérisé par le fait que** la partie de maintien (4) est reliée au boîtier (6) par l'intermédiaire d'une liaison à pivot (36).

11. Dispositif (2) selon la revendication 10, **caractérisé par le fait que** la partie de maintien (4) comprend des pivots (34) qui sont enveloppés de matière plastique.

12. Dispositif (2) selon la revendication 10 et/ou la revendication 11, **caractérisé par le fait que** la liaison à pivot (36) comprend des creux d'engagement (38) sur le boîtier (6) qui agissent de concert avec des pivots (34) sur la partie de maintien (4) afin de former ladite liaison à pivot (36).

13. Dispositif (2) selon l'une quelconque des revendications précédentes, **caractérisé par le fait que** la partie de maintien (4) peut être détachée du boîtier (6) par une légère déformation élastique réversible.

14. Dispositif (2) selon l'une quelconque des revendications précédentes, **caractérisé par le fait que** la partie de maintien (4) est réalisée du type arceau de telle sorte que le boîtier (6) s'engage dans l'ouverture traversante (33) formée par la partie de maintien (4) ou par la partie de maintien (4) et le boîtier (6), lorsque la partie de maintien (4) se trouve dans la position repliée (S) .

15. Dispositif présentant les caractéristiques du préambule de la revendication 1, **caractérisé par** une partie de maintien de forme stable destinée à fixer le dispositif sur un support, en particulier une entretoise de lit, une tige et/ou un cadre, laquelle partie de maintien peut pivoter par rapport au boîtier entre une position d'utilisation dans laquelle elle fait saillie au moins en partie du boîtier et une position repliée dans laquelle elle est au moins en partie, de préférence le long de l'ensemble de son extension, en appui sur le boîtier, et **caractérisé en outre par le fait que** la partie de maintien peut pivoter autour d'un axe de pivotement s'étendant horizontalement dans une position de fonctionnement normale du dispositif, entre la position d'utilisation et la position repliée, la partie de maintien (4) ayant une portion de type crochet (28) pour un montage accrochant et/ou agrafant du dispositif (2) sur le support (30).
